# EUROPÄISCHE PATENTANMELDUNG

(11) **EP 4 775 248 A1**
(43) Veröffentlichungstag der Anmeldung: **15.07.2026**
(21) Anmeldenummer: 26151701.5
(22) Anmeldetag: 13.01.2026
(51) Int. Cl.: A61M 16/10, A61M 16/00, A61M 16/08, A61M 16/12

(54) **VORRICHTUNG ZUM DOSIEREN EINES STICKSTOFFMONOXID ENTHALTENDEN THERAPIEGASES**

(30) Priorität: 13.01.2025 DE 102025100961
(71) Anmelder: EKU Elektronik GmbH, 56291 Leiningen (DE)
(72) Erfinder: Köbrich, Dr. Rainer, 35321 Laubach (DE); Stemann, Daniel, 33469 Simmern (DE)
(74) Vertreter: Münzel, Joachim R.

(57) **Zusammenfassung**

Eine erfindungsgemäße Vorrichtung zur Verabreichung von Stickstoffmonoxid an einen Patienten weist eine Spülgasausleitung (10) auf, die über eine Verbindungsleitung mit der Raumluftzuleitung (13) eines Raumluftanalysators (12) strömungsverbunden ist. In einem Betriebszustand der Vorrichtung wird ein definierter Therapiegasstrom in den durch die Raumluftzuleitung (13) geführten Raumluftgasstrom eingeleitet und in dem entstehenden Mischgasstrom die Stickstoffmonoxid-Konzentration ermittelt. Aus dem so ermittelten Wert kann eine Information darüber gewonnen werden, ob die NO-Konzentration eines Therapiegases, das in einem an die Vorrichtung angeschlossenen Druckgasbehälter (6) enthalten ist, einem für die Therapie vorgesehenen Wert tatsächlich entspricht.

## Beschreibung

Die Erfindung betrifft eine Vorrichtung zum Dosieren eines Stickstoffmonoxid (NO) enthaltenden Therapiegases, mit einer Dosierleitung, die mit einem Behälter für ein Stickstoffmonoxid enthaltendes Therapiegas sowie mit einer Therapiegaszuleitung zum Einspeisen von Therapiegas aus dem Behälter in das Atemgas eines Patienten verbindbar und mit einer Durchfluss-Regeleinrichtung zum Einstellen eines definierten Gasflusses durch die Dosierleitung ausgerüstet ist, mit einer von der Dosierleitung abzweigenden Spülgasausleitung zum Abführen eines Spülgasstroms, mit einer ersten Ventilanordnung zum Einstellen eines über die Spülgasausleitung und/oder die Therapiegaszuleitung geführten Gasstroms, mit einer Steuereinheit zum Ansteuern der Durchfluss-Regeleinrichtung und/oder der ersten Ventilanordnung und mit einem Raumluftanalysator, der mit einer Raumluftzuleitung, einer in der Raumluftzuleitung angeordneten Fördereinrichtung zum Durchleiten eines definierten Raumluftgasstroms durch die Raumluftzuleitung und mit einer Messeinrichtung zum Erfassen der Konzentration von Stickstoffmonoxid im Raumluftgasstrom ausgerüstet ist.

Vorrichtungen zum Dosieren eines Stickstoffmonoxid enthaltenden Therapiegases, beispielsweise für die inhalative Stickstoffmonoxid-Therapie (iNO-Therapie), sind etwa aus der US 2015 035 2311 A1, der US 6 125 846 A1, der EP 2 967 434 A1, der EP 4 316 555 A2 oder der DE 10 2010 016 699 A1 bekannt. Derartige Vorrichtungen ermöglichen die Einspeisung eines genau definierten Therapiegasstroms in das Atemgas eines Patienten. Da sich Stickstoffmonoxid mit der Zeit zu schädlichem Stickstoffdioxid (NO₂) umsetzt, sind derartige Vorrichtungen zumeist mit Spülsystemen ausgestattet, die es ermöglichen, die Rohrleitungen in der Dosiervorrichtung in regelmäßigen Abständen, zumindest aber nach längeren Standzeiten und/oder vor Beginn eines Einsatzes, mit frischem Therapiegas durchzuspülen und so das residuale Volumen in die Umwelt abzuführen.

Weiterhin wird im klinischen Bereich aus Gründen des Arbeitsschutzes zunehmend die Verwendung von Raumluft-Analysatoren verlangt. Bei der iNO-Therapie zum Einsatz kommende Raumluft-Analysatoren sprechen sehr empfindlich insbesondere auf Stickstoffmonoxid an und können beispielsweise Konzentrationen von NO in der Umgebungsluft zwischen 0 und 100 ppm (V/V) sehr genau erfassen. Raumluft-Analysatoren werden zunehmend in die am Markt vertriebenen Dosiergeräte eingebaut; beispielsweise vertreibt die Anmelderin unter der Bezeichnung NO-A ein Dosiergerät für die inhalative Stickstoffmonoxid-Therapie (iNO), in welchem ein derartiger Raumluftanalysator integriert ist.

Stickstoffmonoxid enthaltende Therapiegase werden von verschiedenen Herstellern mit unterschiedlichen Konzentrationen angeboten. Üblich und beispielsweise in der Europäischen Union zur Verwendung als Therapiegas zugelassen sind beispielsweise Gasgemische, die aus einem Volumenanteil zwischen 450 ppm und 1000 ppm Stickstoffmonoxid, Rest Stickstoff bestehen. Die Gasgemische werden in Druckgasflaschen bereitgestellt, die vor Beginn einer Therapie an die Dosiervorrichtung angeschlossen werden und nach ihrer Entleerung jeweils ausgetauscht werden müssen. Die jeweils zum Einsatz kommende NO-Konzentration wird den Angaben auf der Druckgasflasche entnommen und in der Regel händisch in die Steuerung der Dosiervorrichtung eingegeben, die daraus den für die jeweilige Therapie erforderlichen Gasfluss ermittelt. Dabei besteht jedoch die Gefahr, dass, beispielsweise aufgrund einer falschen Eingabe und/oder einer fehlerhaften Kennzeichnung der Druckgasflasche, die tatsächliche Therapiegaskonzentration im Behälter von der Einstellung der Dosiervorrichtung abweicht. Ein besonders kritischer Zeitpunkt besteht insbesondere beim Austausch der Druckgasflasche: Wird versehentlich eine Druckgasflasche mit einer veränderten NO-Konzentration angeschlossen und die Einstellung des Gasflusses in der Dosiervorrichtung nicht angepasst und/oder weicht die tatsächliche NO-Konzentration in der Druckgasflasche von der angegebenen Konzentration ab, kommt es zu einer Fehlversorgung des Patienten, die für diesen lebensbedrohlich sein kann.

Der Erfindung liegt daher die Aufgabe zu Grunde, eine Vorrichtung zur Dosierung eines Stickstoffmonoxid enthaltenden Therapiegases an einen Patienten zu entwickeln, bei der die Gefahr einer Fehlversorgung des Patienten aufgrund einer fehlerhaften Einstellung des Gasflusses minimiert ist.

Gelöst ist diese Aufgabe durch eine Vorrichtung mit den Merkmalen des Patentanspruchs 1 sowie durch ein bei einer solchen Vorrichtung ablaufendes Verfahren mit den Merkmalen des Patentanspruchs 5. Vorteilhafte Ausgestaltungen der Erfindung sind in den Unteransprüchen angegeben.

Eine erfindungsgemäße Vorrichtung der eingangs genannten Art und Zweckbestimmung ist also dadurch gekennzeichnet, dass die Spülgasausleitung mit der Raumluftzuleitung des Raumluftanalysators über eine mit einer zweiten Ventilanordnung zum Einstellen eines Gasstroms ausgerüstete Verbindungsleitung strömungsverbunden ist und die Steuereinheit so eingerichtet ist, dass in einem Betriebszustand der Vorrichtung ein definierter Therapiegasstrom aus dem Behälter über die Verbindungsleitung in den durch die Raumluftzuleitung geführten Raumluftgasstrom eingeleitet wird.

Die erfindungsgemäße Vorrichtung verfügt über einen Raumluft-Analysator, der bevorzugt so ausgebildet ist, dass er eine Konzentration zwischen 0 und 100 ppm Stickstoffmonoxid im Volumen eines den Raumluft-Analysator durchströmenden Gasstroms sehr genau ermitteln kann. Er kann weiterhin so ausgebildet sein, dass er weitere Gase im Raumluftstrom, insbesondere NO₂, detektieren kann.

Die erfindungsgemäße Vorrichtung weist darüber hinaus eine Spülgasausleitung auf, über die eine Spülung der Vorrichtung erfolgen kann. Beim Spülvorgang wird, manuell oder nach einem in der Steuereinheit gespeicherten Programm, ein begrenzter Mengenstrom an Therapiegas (Spülgasstrom) durch die Dosierleitung und die Spülgasausleitung geleitet, wodurch unerwünschte Gaskomponenten, wie NO₂, die sich in der Dosierleitung angesammelt haben, ausgespült werden.

Erfindungsgemäß besteht über die Verbindungsleitung eine Strömungsverbindung zwischen der Spülgasausleitung und der Raumluftzuleitung des Raumluft-Analysators. Diese Strömungsverbindung ermöglicht es, einen definierten Therapiegasstrom aus einem an die erfindungsgemäße Vorrichtung angeschlossenen Behälter, beispielsweise einer Druckgasflasche, in den durch die Raumluftzuleitung geführten Raumluftstrom einzuleiten und auf diese Weise in der Raumluftzuleitung ein Mischgas zu erzeugen, das eine gegenüber dem Therapiegas in der Druckgasflasche verringerte Konzentration an Stickstoffmonoxid aufweist. Durch eine geeignete Wahl des Verhältnisses der Volumenströme der beiden miteinander vermischten Gasströme liegt die Stickstoffmonoxid-Konzentration im Mischgas innerhalb des Messbereiches der Messeinrichtung des Raumluft-Analysators. Dadurch kann die Stickstoffmonoxid-Konzentration im Mischgas genau bestimmt werden, wodurch zugleich eine Information über die NO-Konzentration des Therapiegases gewonnen ist. Übliche Messeinrichtungen von Raumluftanalysatoren sind beispielsweise in der Lage, eine Konzentration von Stickstoffmonoxid in einen Bereich von 0 bis 100 ppm genau erfassen. Ist somit im Therapiegas ein Anteil zwischen 400 ppm und 1000 ppm NO enthalten, kann die Messeinrichtung die NO-Konzentration im Mischgas genau bestimmen, wenn das Therapiegas durch die Raumluft mindestens im Verhältnis 1:10 verdünnt wird. Ein bevorzugtes Mischungsverhältnis von Therapiegas und Raumluft beträgt daher zwischen 1:10 und 1:20.

In der Steuereinheit ist wenigstens ein elektronisches Steuerprogramm eingespeichert, mittels dessen verschiedene Betriebszustände der erfindungsgemäßen Vorrichtung eingestellt werden können. Beispielsweise läuft in einem ersten Betriebszustand ein hier als "Therapieprogramm" bezeichnetes Steuerprogramm in der Steuereinheit ab, bei dem eine Zuführung eines Therapiegasstroms aus dem Behälter über die Dosierleitung und die Therapiegasleitung an einen Patienten nach einem im Therapieprogramm vorgegebenen Ablauf erfolgt. Zumindest während der Zuführung des Therapiegases an den Patienten ist auch der Raumluft-Analysator in Betrieb und ermittelt mögliche Spuren von Stickstoffmonoxid und ggf. anderen Gasen in der Atmosphäre des Behandlungsraums.

In einem zweiten Betriebszustand kann ein hier als "Spülprogramm" bezeichnetes Steuerprogramm ausgeführt werden, das eine Spülung in der oben beschriebenen Weise bewirkt und die Dosierleitung von residualen Spuren von Stickstoffdioxid befreit. Das Spülgas wird über die Spülgasausleitung, an der Verbindungsleitung vorbei, abgeführt und in die Außenatmosphäre abgegeben oder in einem Behälter aufgefangen.

In einem weiteren Betriebszustand der Vorrichtung läuft in der Steuereinheit ein Steuerprogramm ab, das hier auch als "Gaskontrollprogramm" bezeichnet wird. Dabei wird ein definierter Therapiegasstrom über die Spülgasausleitung und die Verbindungsleitung in den durch die Raumluftzuleitung geführten definierten Raumluftgasstrom eingeleitet, und in dem entstehenden Mischgasstrom wird mittels der Messeinrichtung des Raumluft-Analysators ein Wert für die Stickstoffmonoxid-Konzentration ermittelt.

Als "definierter" Gasstrom soll hier ein Gasstrom bezeichnet werden, dessen Volumenstrom möglichst genau bekannt ist. Ein "definierter Raumluftstrom" wird im Raumluft-Analysator dadurch hergestellt, dass die Fördereinrichtung im Raumluft-Analysator auf einen bestimmten Wert für den Gasdurchfluss eingestellt wird. Ein "definierter Therapiegasstrom" kann im Gaskontrollprogramm mittels der Durchfluss-Regeleinrichtung in der Dosiergasleitung hergestellt werden oder durch eine Ansteuerung der ersten und/oder der zweiten Ventilanordnung. Im letzteren Fall kann die Ansteuerung auch derart erfolgen, dass die Strömungsverbindung über die Spülgasausleitung und die Verbindungsleitung in einer genau definierten Weise intermittierend geöffnet und wieder geschlossen wird. Durch die Einstellung exakter Öffnungs- und Schließdauern der Ventilanordnung(en) kann sehr genau ein mittlerer Volumenstrom des Therapiegases eingestellt werden, der der Raumluftzuleitung zugeführt wird.

Aus der ermittelten NO-Konzentration im Mischgasstrom lässt sich eine Information über die Stickstoffmonoxid-Konzentration im Therapiegas gewinnen. Eine vorteilhafte Ausgestaltung der Erfindung sieht dabei vor, dass die Steuereinheit mit dem Raumluftanalysator in Datenverbindung steht und so eingerichtet ist, dass sie aus einem von der Messeinrichtung ermittelten Wert für die Stickstoffmonoxid-Konzentration im Mischgasstrom einen Wert für die Stickstoffmonoxid-Konzentration im Therapiegas errechnet und diesen auf einer mit der Steuereinheit datenverbundenen Anzeigeeinheit anzeigt. Auf diese Weise besteht die Möglichkeit festzustellen, ob der ermittelte Wert der NO-Konzentration einem in der Steuereinheit eingegebenen oder auf dem Behälter vermerkten Wert entspricht.

Ist umgekehrt der Wert für die Stickstoffmonoxid-Konzentration im Behälter genau bekannt, beispielsweise aufgrund eines Analysezertifikats des Gaselieferanten, kann die Messung der NO-Konzentration im Mischgas jedoch auch zur Kalibrierung des Raumluftanalysators verwendet werden.

Zweckmäßigerweise steht die Steuereinrichtung mit einem Signalgeber in Datenverbindung, der bei Überschreiten einer vorgegebenen Abweichung des errechneten Werts für die Stickstoffmonoxid-Konzentration von einem eingestellten und/oder in der Steuereinheit eingespeicherten Wert ein Warnsignal abgibt. Alternativ oder ergänzend zu einem Signalgeber kann ein Warnsignal auch auf einer mit der Steuereinheit datenverbundenen Anzeigeeinheit angezeigt werden.

Eine gleichfalls vorteilhafte Weiterbildung der Erfindung sieht vor, dass die Messeinrichtung des Raumluft-Analysators für die Messung einer Stickstoffdioxid (NO₂)-Konzentration geeignet ist. Bei der Vermischung des Therapiegases mit der durch den Raumluftanalysator geleiteten Raumluft stellt sich nach einer gewissen Zeit eine konstante NO₂ - Konzentration im Mischgasstrom ein, die von der Stickstoffmonoxid-Konzentration im Therapiegas abhängt. Ist diese genau bekannt, beispielsweise bei Vorliegen eines Analysezertifikats, kann auf diese Weise ein Wert für die Stickstoffdioxid-Konzentration errechnet, mit dem von der Messeinrichtung ermittelten Wert verglichen und somit die Funktionsfähigkeit der Messeinrichtung überprüft werden.

Ein bevorzugtes Verfahren zur Gaskontrolle bei einer erfindungsgemäßen Vorrichtung ist dadurch gekennzeichnet, dass
- ein definierter Therapiegasstrom aus einem an die Dosierleitung angeschlossenen Behälter über die Spülgasausleitung und die Verbindungsleitung in die Raumluftzuleitung eingeleitet und dort mit einem definierten Raumluftgasstrom zu einem Mischgasstrom vermischt wird,
- mittels der Messeinrichtung des Raumluftanalysators eine Konzentration von Stickstoffmonoxid im Mischgasstrom erfasst wird,
- aus der erfassten Konzentration von Stickstoffmonoxid im Mischgasstrom in der Steuereinheit ein Wert für die Konzentration von Stickstoffmonoxid im Therapiegasstrom errechnet wird,
- die Steuereinheit den errechneten Wert mit einem in der Steuereinheit eingegebenen Wert für die Konzentration von Stickstoffmonoxid im Behälter vergleicht und eine Information über den Vergleich der beiden Werte an einer mit der Steuereinheit datenverbundenen Anzeigeeinheit visualisiert wird.

Das erfindungsgemäße Verfahren, hier auch "Gaskontrollprogramm" genannt, dient insbesondere zwei Zwecken: Zum einen kann die Information über den Vergleich der beiden Werte für die Stickstoffmonoxid-Konzentration dazu genutzt werden, den Inhalt des angeschlossenen Behälters zu überprüfen. Zum zweiten ermöglicht das Verfahren bei genau bekannter Zusammensetzung des Gases im angeschlossenen Behälter, die Messeinrichtung des Raumluft-Analysators zu kontrollieren und ggf. neu zu kalibrieren. Die Information über den Vergleich der beiden Werte wird beispielsweise auf einem Bildschirm angezeigt.

Der dem Raumluftanalysator zugeführte definierte Therapiegasstrom wird bevorzugt dadurch erzeugt, dass die Strömungsverbindung über die Spülgasausleitung und die Verbindungsleitung in einer genau definierten Weise intermittierend geöffnet und wieder geschlossen wird, wodurch ein mittlerer Volumenstrom des Therapiegases sehr genau eingestellt werden kann.

Das Verhältnis der Volumenströme aus Therapiegasstrom und Raumluftgasstrom in der Raumluftzuleitung muss so gewählt werden, dass die NO-Konzentration im Mischgasstrom innerhalb des Messbereichs der Messeinrichtung des Raumluft-Analysators liegt. Übliche iNO-Therapiegase besitzen einen Anteil zwischen 400 und 1000 ppm Stickstoffmonoxid. Liegt der Messbereich der Messeinrichtung zwischen 0 und 100ppm, empfiehlt sich daher für das Verhältnis der Volumenströme aus Therapiegasstrom und Raumluftgasstrom ein Wert zwischen 1:10 und 1:20.

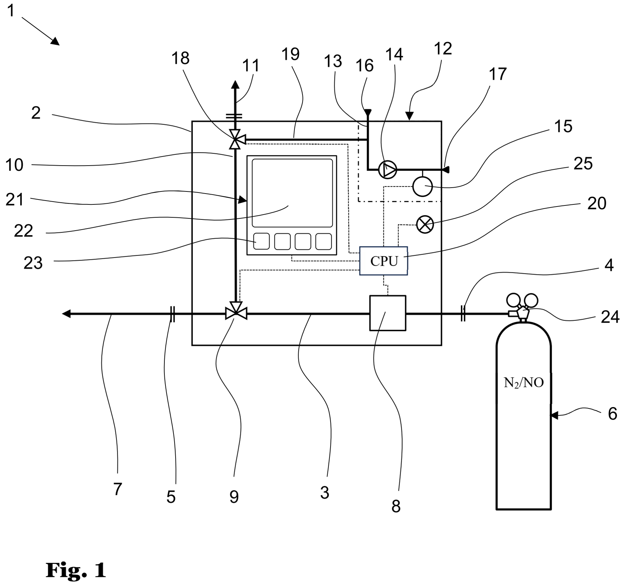

Anhand der Zeichnung soll ein Ausführungsbeispiel der Erfindung näher erläutert werden. Die einzige Zeichnung (Fig. 1) zeigt schematisch eine erfindungsgemäße Vorrichtung in einem Blockschaltbild.

Die in der Zeichnung gezeigte Vorrichtung 1 umfasst ein Gehäuse 2, durch das eine Dosierleitung 3 hindurchgeführt ist. Die Dosierleitung 3 endet außerhalb des Gehäuses 2 jeweils an Anschlussstutzen 4, 5. Der Anschlussstutzen 4 dient zum Anschließen eines mit einem Therapiegas gefüllten Behälters, beispielsweise einer Druckgasflasche 6. Als Therapiegas kommt erfindungsgemäß ein Stickstoffmonoxid enthaltendes Gas zum Einsatz, beispielsweise eine aus Stickstoff und Stickstoffmonoxid bestehende Gasmischung mit einem NO-Anteil zwischen 400 ppm und 1000 ppm. Der Anschlussstutzen 5 dient zum Anschließen einer Therapiegaszuleitung 7, die mit einer hier nicht gezeigten Atemgaszuleitung eines Patienten strömungsverbunden ist. In der Dosierleitung 3 ist eine Durchfluss-Regeleinrichtung 8 angeordnet, die für einen definierten Volumenstrom des durch die Dosierleitung 3 geführten Therapiegases sorgt.

Stromab zur Durchfluss-Regeleinrichtung 8 zweigt an einer ersten Ventilanordnung 9 eine Spülgasausleitung 10 von der Dosierleitung 3 ab. Bei der Ventilanordnung 9 handelt es sich im hier gezeigten Beispiel um ein Dreiwegeventil, jedoch sind separate Ventile in den Leitungen 3, 10 ebenfalls vorstellbar. An einem Anschlussstutzen der Spülgasausleitung 10 ist eine Abgasleitung 11 zum Wegführen des Spülgases angeschlossen.

Die Vorrichtung 1 verfügt des Weiteren über einen im Gehäuse 2 integrierten Raumluft-Analysator 12. Der Raumluft-Analysator 12 weist eine Raumluft-Zuleitung 13 auf, in der eine Fördereinrichtung 14, beispielsweise ein Pumpe, integriert ist. In Förderrichtung stromab zu dieser ist in der Raumluft-Zuleitung 13 eine Messeinrichtung 15 angeordnet. Die Messeinrichtung 15 eignet sich dazu, eine NO-Konzentration in der von der Fördereinrichtung 14 angesaugten Raumluft in einem Bereich von beispielsweise zwischen 0 und 100 ppm detektieren zu können; darüber hinaus kann sie optional auch dazu geeignet sein, weitere Gase in der zugeführten Raumluft erfassen zu können, wie beispielsweise NO₂. Die Raumluft-Zuleitung 13 beginnt an einem Gaseintritt 16 und mündet an einem Gasauslass 17 aus dem Gehäuse 2 aus.

Von der Spülgasleitung 10 zweigt an einer zweiten Ventilanordnung 18, bei der es sich im hier gezeigten Ausführungsbeispiel wiederum um ein Dreiwegeventil handelt, eine Verbindungsleitung 19 ab, die in die Raumluft-Zuleitung 13 einmündet.

Die Durchfluss-Regeleinrichtung 8, die Ventilanordnungen 9, 18 und die Messeinrichtung 15 sind elektronisch steuerbar und stehen mit einer Steuereinheit 20 in Datenverbindung, die ihrerseits mit einer Anzeige- und Bedieneinheit 21 datenverbunden ist. In der elektronischen Steuereinheit 20 sind verschiedene Datenverarbeitungsprogramme (Steuerprogramme) eingespeichert, mittels denen die Durchfluss-Regeleinrichtung 8 und die Ventilanordnungen 9, 18 in der unten näher beschriebenen Weise angesteuert werden können. Die Anzeige- und Bedieneinheit 21 weist einen Bildschirm 22 zur Anzeige von Informationen auf; beispielsweise kann am Bildschirm 22 das laufende Steuerprogramm, die Größe eines durch die Dosierleitung 3 geführten Therapiegas-Volumenstroms oder eine an der Messeinrichtung 15 gemessene NO-Konzentration angezeigt werden. Weiterhin ist die Anzeige- und Bedieneinheit mit einem Eingabepanel 23 ausgestattet, über das beispielsweise ein auszuwählendes Steuerprogramm, die NO-Konzentration des in der Gasflasche 6 befindlichen Therapiegases und/oder Angaben über die jeweilige Therapie oder Patientendaten eingegeben werden können. Bildschirm 22 und Eingabepanel 23 können im Übrigen auch in einem Touchscreen integriert sein.

Im Übrigen kann die Vorrichtung 1 darüber hinaus mit weiteren Bauteilen ausgestattet sein, wie beispielsweise einer Regeleinrichtung zum Regeln des Therapiegasstroms in Abhängigkeit von gemessenen Parametern, wie beispielsweise einer NO-Konzentration im Atemgas eines Patienten; derartige Bauteile sind aber für das Verständnis der vorliegenden Erfindung ohne Relevanz und daher aus Gründen der Übersichtlichkeit hier nicht gezeigt.

Im Betrieb der Vorrichtung 1 wird die mit einem Druckreduzierventil 24 ausgerüstete Druckgasflasche 6 an den Anschlussstutzen 4 in an sich bekannter Weise angeschlossen und ein an der Druckgasflasche 6 vorhandenes Druckreduzierventil 24 auf einen vorgegebenen Maximaldruck von beispielsweise 4 bar - zur Erzeugung eines Medikamentenstroms von bis zu 12 I/min - eingestellt. Für den weiteren Betrieb stehen in der Steuereinheit 20 verschiedene im folgenden erläuterte Steuerprogramme zur Verfügung, die am Bedienpanel 23 durch einen Therapeuten ausgewählt werden können.

In einem ersten, hier als "Therapieprogramm" bezeichneten Steuerprogramm wird eine Strömungsverbindung von der Druckgasflasche 6 über die Dosierleitung 3 zur Therapiegasleitung 7 hergestellt, die in hier nicht gezeigter Weise mit einer Atemgaszuleitung eines Patienten strömungsverbunden ist. Die Strömungsverbindung zur Spülgasausleitung 10 ist geschlossen. Bei Ablauf dieses Programms wird dem Patienten ein definierter Therapiegasstrom zugeführt. An der Anzeige- und Bedieneinheit 21 werden vom Therapeuten weitere, für die jeweilige Therapie relevante Parameter eingegeben; dazu gehört insbesondere die Zielkonzentration des dem Patienten zuzuführenden Therapiegases, der wiederum abhängt von der Stickstoffmonoxid-Konzentration des Therapiegases in der Druckgasflasche 6. Der Raumluft-Analysator 12 ist in Betrieb, d.h. ein definierter Volumenstrom an Raumluft von beispielsweise 200 ml/min wird zum Messgerät 15 geführt, welches daraus die Konzentration des darin enthaltenen Stickstoffmonoxids (und gegebenenfalls weiterer Gase) ermittelt. Der von der Messeinrichtung 15 ermittelte Wert kann am Bildschirm 22 visualisiert werden.

Ein weiteres, hier als "Spülprogramm" gezeichnetes Steuerprogramm kommt insbesondere nach längeren Standzeiten zum Einsatz, wenn beispielsweise die Gefahr besteht, dass sich in der Dosierleitung von der letzten Therapie noch verbliebenes Stickstoffmonoxid ganz oder teilweise in schädliches Stickstoffdioxid (NO₂) umgesetzt hat. Beim Starten des Spülprogramms wird die Strömungsverbindung zwischen Dosierleitung 3 und Therapiegasleitung 7 an der Ventilanordnung 9 geschlossen und eine Strömungsverbindung zwischen Dosierleitung 3 und Spülgasausleitung 10 hergestellt. Die Strömungsverbindung zwischen Spülgasausleitung 10 und Verbindungsleitung 19 ist geschlossen. Während des Spülprogramms wird für einen vorgegebenen Zeitraum ein Gasstrom (Spülgasstrom) aus der Druckgasflasche 6 über die Dosierleitung 3 und die Spülgasleitung 10 und von dort in die Abgasleitung 11 geleitet. Der Spülgasstrom wird anschließend in hier nicht gezeigter Weise aufgefangen oder außerhalb des Behandlungsraumes in die Umgebungsluft entlassen.

In einem dritten Steuerprogramm, hier als "Gaskontrollprogramm" bezeichnet, erfolgt eine Kontrolle der Stickstoffmonoxid-Konzentration des Therapiegases aus der Druckgasflasche 6. Beim Starten des Gaskontrollprogramms wird die Strömungsverbindung zwischen Dosierleitung 3 und Therapiegasleitung 7 an der Ventilanordnung 9 geschlossen und eine Strömungsverbindung zwischen Dosierleitung 3, der Spülgasausleitung 10 und der Verbindungsleitung 19 hergestellt. Die Strömungsverbindung zur Abgasleitung 11 wird geschlossen. Der Raumluft-Analysator 12 ist in Betrieb und führt einen Raumluftstrom von beispielsweise 200 ml/min durch die Raumluftzuleitung 13 hindurch. Zugleich wird ein definierter Volumenstrom von beispielsweise 20 ml/min Therapiegas über die Dosierleitung 3, die Spülgasausleitung 10 und die Verbindungsleitung 19 geführt und in die Raumluftzuleitung 13 geleitet. Die Volumenstrom des Therapiegases kann dabei durch eine Regelung an der Durchfluss-Regeleinrichtung 8 eingestellt werden; alternativ kann durch eine vorgegebene Abfolge von Schaltungen der Ventilanordnung 9 und/oder der Ventilanordnung 18 eine intermittierender Eintrag von Therapiegas in die Raumluftzuleitung 13 erfolgen, der einen gut definierten Mittelwert für den Therapiegasstrom bewirkt.

In der Raumluftzuleitung 13 vermischt sich der Therapiegasstrom aus der Verbindungsleitung 19 mit dem Raumluftstrom. An der Messeinrichtung 15 wird daher die NO-Konzentration im Mischgasstrom gemessen. Aufgrund der exakt vorgegebenen Strömungsverhältnisse kann die Steuereinheit 20 aus dem gemessenen Wert einen genauen Wert für die Konzentration des Therapiegases in der Druckgasflasche 6 zurückberechnen. Da der Therapiegasstrom durch den Raumluftstrom verdünnt wird - im hier genannten Beispiel im Verhältnis 1:10 - kann die Stickstoffmonoxid-Konzentration auch dann genau bestimmt werden, wenn die NO-Konzentration in der Druckgasflasche 6, beispielsweise 500 ppm, wesentlich höher ist als der Messbereich das Messgeräts 15, beispielsweise 0-100 ppm.

Der zurückberechnete Wert für die NO-Konzentration des Therapiegases in der Druckgasflasche 6 kann in der Steuereinheit 20 mit einem vorgegebenen Wert verglichen werden. Wird hierbei eine Abweichung festgestellt, die oberhalb eines vorgegebenen Toleranzbereichs liegt, wird eine Fehlermeldung abgegeben, die beispielsweise auf dem Bildschirm 22 angezeigt und/oder durch einen mit der Steuereinheit 20 datenverbundenen Signalgeber 25, beispielsweise einer Warnleuchte, visualisiert wird. Die Fehlermeldung zeigt dem Therapeuten an, dass die Stickstoffmonoxid-Konzentration in der Druckgasflasche 6 nicht dem im Steuerprogramm eingegebenen Wert entspricht. Das Gaskontrollprogramm kommt insbesondere nach dem Wechsel einer Druckgasflasche 7 während einer laufenden Therapie zum Einsatz, wenn die Gefahr besteht, dass versehentlich eine Druckgasflasche 7 mit einer abweichenden NO-Konzentration angeschlossen wurde oder die tatsächliche NO-Konzentration in der Druckgasflasche nicht einem auf der Druckgasflasche 7 angegebenen Wert entspricht.

Umgekehrt kann das Gaskontrollprogramm bei bekannter Stickstoffmonoxid-Konzentration in der Druckgasflasche 6, etwa bei Vorliegen eines Analysezertifikats des Gaselieferanten, dazu genutzt werden, die Messeinrichtung 15 zu überprüfen und ggf. zu neu zu kalibrieren. Eine derartige Überprüfung sollte in regelmäßigen Zeitabständen durchgeführt werden, um die Zuverlässigkeit der Vorrichtung 1 dauerhaft zu gewährleisten.

### Bezugszeichenliste:

- 1: Vorrichtung
- 2: Gehäuse
- 3: Dosierleitung
- 4: Anschlussstutzen
- 5: Anschlussstutzen
- 6: Druckgasflasche
- 7: Therapiegaszuleitung
- 8: Durchfluss-Regeleinrichtung
- 9: Ventilanordnung
- 10: Spülgasausleitung
- 11: Abgasleitung
- 12: Raumluft-Analysator
- 13: Raumluft-Zuleitung
- 14: Fördereinrichtung
- 15: Messeinrichtung
- 16: Gaseintritt
- 17: Gasauslass
- 18: Ventilanordnung
- 19: Verbindungsleitung
- 20: Steuereinheit
- 21: Anzeige- und Bedieneinheit
- 22: Bildschirm
- 23: Eingabepanel
- 24: Druckreduzierventil
- 25: Warnleuchte

## Patentansprüche

1. Vorrichtung zum Dosieren eines Stickstoffmonoxid enthaltenden Therapiegases, mit einer Dosierleitung (3), die mit einem Behälter (6) für ein Stickstoffmonoxid enthaltendes Therapiegas sowie mit einer Therapiegaszuleitung (7) zum Einspeisen von Therapiegas aus dem Behälter (6) in das Atemgas eines Patienten verbindbar und mit einer Durchfluss-Regeleinrichtung (8) zum Einstellen eines definierten Gasflusses durch die Dosierleitung (3) ausgerüstet ist, mit einer von der Dosierleitung (3) abzweigenden Spülgasausleitung (10) zum Abführen eines Spülgasstroms, mit einer ersten Ventilanordnung (9) zum Einstellen eines über die Spülgasausleitung (10) und/oder die Therapiegaszuleitung (7) geführten Gasstroms, mit einer Steuereinheit (20) zum Ansteuern der Durchfluss-Regeleinrichtung (8) und/oder der ersten Ventilanordnung (9), und mit einem Raumluftanalysator (12), der mit einer Raumluftzuleitung (13), einer in der Raumluftzuleitung angeordneten Fördereinrichtung (14) zum Durchleiten eines definierten Raumluftgasstroms durch die Raumluftzuleitung (13) und mit einer Messeinrichtung (15) zum Erfassen der Konzentration von Stickstoffmonoxid im Raumluftgasstrom ausgerüstet ist,
**dadurch gekennzeichnet,**
**dass** die Spülgasausleitung (10) mit der Raumluftzuleitung (13) des Raumluftanalysators (12) über eine mit einer zweiten Ventilanordnung (18) zum Einstellen eines Gasstroms ausgerüsteten Verbindungsleitung (19) strömungsverbunden ist und die Steuereinheit (20) so eingerichtet ist, dass in einem Betriebszustand der Vorrichtung (1) ein definierter Therapiegasstrom aus dem Behälter (6) über die Verbindungsleitung (19) in den durch die Raumluftzuleitung (13) geführten Raumluftgasstrom eingeleitet wird.

2. Vorrichtung nach Anspruch 1, **dadurch gekennzeichnet, dass** die Steuereinheit (20) mit dem Raumluftanalysator (12) in Datenverbindung steht und so eingerichtet ist, dass sie aus einem von der Messeinrichtung (15) ermittelten Wert für die Stickstoffmonoxid-Konzentration in dem mit dem Therapiegasstrom vermischten Raumluftstrom einen Wert für die Stickstoffmonoxid-Konzentration im Therapiegas errechnet.

3. Vorrichtung nach Anspruch 2, **dadurch gekennzeichnet, dass** die Steuereinrichtung (20) mit einem Signalgeber (25) in Datenverbindung steht, der bei Überschreiten einer vorgegebenen minimalen Abweichung des errechneten Werts für die Stickstoffmonoxid-Konzentration von einem eingestellten Wert ein Warnsignal abgibt.

4. Vorrichtung nach einem der vorhergehenden Ansprüche, **dadurch gekennzeichnet, dass**, die Messeinrichtung (15) zum Erfassen der Konzentration von Stickstoffdioxid im Raumluftgasstrom geeignet ist.

5. Verfahren zur Gaskontrolle bei einer Vorrichtung nach einem der vorhergehenden Ansprüche, bei dem
- ein definierter Therapiegasstrom aus einem an die Dosierleitung (3) angeschlossenen Behälter (6) über die Spülgasausleitung (10) und die Verbindungsleitung (19) in die Raumluftzuleitung (13) eingeleitet und dort mit einem definierten Raumluftgasstrom zu einem Mischgasstrom vermischt wird,
- mittels der Messeinrichtung (15) des Raumluftanalysators (12) eine Konzentration von Stickstoffmonoxid im Mischgasstrom erfasst wird,
- aus der erfassten Konzentration von Stickstoffmonoxid im Mischgasstrom in der Steuereinheit (20) ein Wert der Konzentration von Stickstoffmonoxid im Therapiegasstrom errechnet wird,
- die Steuereinheit (20) den errechneten Wert mit einem in der Steuereinheit (20) eingegebenen Wert für die Konzentration von Stickstoffmonoxid im Behälter (6) vergleicht und eine Information über den Vergleich der beiden Werte an einer mit der Steuereinheit (20) datenverbundenen Anzeigeeinheit (21) visualisiert wird.

6. Verfahren nach Anspruch 5, **dadurch gekennzeichnet, dass** der dem Raumluftanalysator (12) zugeführte definierte Therapiegasstrom durch eine intermittierende Schaltung der ersten Ventilanordnung (9) hergestellt wird.

7. Verfahren nach Anspruch 5 oder 6, **dadurch gekennzeichnet, dass** das Verhältnis der Volumenströme von Therapiegasstrom zu Raumluftstrom in der Raumluftzuleitung (13) zwischen 1:10 und 1:20 beträgt
